# EUROPEAN PATENT SPECIFICATION

(11) **EP 2 391 326 B1**
(45) Date of publication and mention of the grant of the patent: **30.07.2014**
(21) Application number: 10701259.3
(22) Date of filing: 21.01.2010
(51) Int. Cl.: A61F 15/00, A61F 5/01, A61F 5/02

(54) **IMPROVEMENTS IN PELVIC SUPPORT**
VERBESSERUNGEN VON BECKENSTÜTZVORRICHTUNGEN
AMÉLIORATIONS APPORTÉES À UN SUPPORT PELVIEN

(30) Priority: 31.01.2009 GB 0901595; 19.08.2009 GB 0914448
(43) Date of publication of application: 07.12.2011
(73) Proprietor: Minos Solutions Limited, Oadby, Leicester (GB)
(72) Inventor: LEWIS, Simon, Enderby Leicester LE19 2RP (GB)
(74) Representative: Oxley, Robin John George
(86) International application number: PCT/GB2010/000093
(87) International publication number: WO 2010/086586

(56) References cited:
- WO-A-97/19658
- GB-A- 2 235 134
- US-A1- 2007 197 945

## Description

This invention relates to improvements in pelvic supports, in particular this invention relates to a tool for assisting in the fitting of a pelvic support.

Pelvic supports are commonly used in the interest of maintaining haemostasis in casualties or patients (hereinafter collectively referred to as casualty) who have suffered from pelvic trauma. Casualties suffering from pelvic trauma or injury, especially in the absence of radiological confirmation of the extent of the trauma or injury, require partial or full pelvic immobilisation to reduce the potential for haemorrhage. This is true irrespective of whether the casualty is inside or outside of a hospital and irrespective of whether the trauma is immediately life threatening or not.

Current medical practice suggests the application of a pelvic support as the optimum patient risk management for a casualty with a suspected pelvic fracture in the pre hospital environment, or indeed in the hospital should a support not have been applied prior to the casualties arrival.

A variety of pelvic supports and pelvic straps are commercially available, each of which requires significant casualty care in the application of the devices. Some examples of these devices such as the Pelvic Binder available from Pelvic Binder, Inc and the SAM Pelvic Sling, available from SAM Medical Products and the disclosure of WO 9719658 in the name of AMBU International, are flexible straps that tighten around the pelvis. Other examples include WO 0189433 of University of New York which pass around the patient and are then partially inflated to provide support. While these products, once fitted to a casualty, provide good pelvic support and significantly decrease the risk of pelvic haemorrhage, there are problems associated with the fitting of such devices.

It is recommended that a casualty suffering from pelvic trauma be moved the minimum amount possible. Pelvic trauma casualties frequently have other internal injuries and moving such a casualty dramatically increases the risk of haemorrhage. The aforementioned pelvic supports, although beneficial once in place, are not easy to apply to the casualty. They are flexible straps that need to be passed around the pelvis. For the majority of casualties this involves passing the support under the casualty which invariably requires the casualty to be lifted or moved contrary to best medical practice.

Further problems associated with present pelvic supports is that they can be very awkward for a single person to fit to a patient and they usually take a period of time to manoeuvre into position and often the patient is moved in a see saw manner as the support is moved into position. Such a motion is particularly bad for disturbing clots that have formed and can aggravate internal bleeding.

The present invention proposes an apparatus and method of attaching a pelvic support that mitigates at least some problems with known pelvic supports.

According to a first aspect of the present invention there is provided a pelvic support application apparatus adapted to position a flexible pelvic support adjacent a patient/casualty and
at least a first rigid or semi-rigid elongate planar element characterised in that it comprises:
a means of releasably attaching a flexible pelvic support adjacent and to said at least one planar element such that, in use the planar element and pelvic support are adapted to be slid between a person and a surface against which the person is supported and wherein the means for attaching the pelvic support comprises two openings in on end of said at least one planar element such that, in use, the pelvic support is adapted to be passed outwardly through one opening, back in the other opening, and back on itself such that it at least partially overlays itself.

By attaching a flexible apparatus to the rigid or semi-rigid first element it can easily be slid under or behind a patient with one swift move, causing minimum disturbance to the pelvic region. Without such a support it can prove very difficult to manoeuvre a flexible pelvic support, frequently being a padded fabric covered strap, under the casualty as, even if the weight of the casualty is supported, the support will often snag on the casualties clothes r the ground.

Preferably the apparatus further comprises a second elongate planar element releasably attachable to the first elongate planar element. The second elongate element may be is rigid or semi-rigid.

In one preferred arrangement the pelvic support is sandwiched between said first and second planar elements.

Preferably the first, and /or the second planar element has a low coefficient of friction. The first and/or second planar element are preferably made of polypropylene.

By providing a semi-rigid low friction surface on either side of the flexible pelvic support, it can easily be slid under a casualty with even less disturbance to the casualties pelvic area.

Preferably the first and/or second planar element has handles at either end thereof. This assists in handling of the apparatus and gives the user something to grab whilst manoeuvring the apparatus and support into position.

Preferably one of the two openings in the end of said first or second planar element also provides the handle.

Preferably the apparatus further comprising attachment means for attaching the first and second planar elements to one another, more preferably the means for attaching the first and second planar elements to one another comprises a multiple hook and loop type fastener. In a preferred embodiment the means for attaching the first and second planar elements to one another is only located at one end of the planar elements.

The apparatus may also include the flexible pelvic support.

In a preferred embodiment the apparatus comprises only a first elongate planar element and the pelvic support, in this embodiment the external face of the pelvic support, when adjacent the planar element comprises a low friction material. In this manner the embodiment with a single planar element comprises low friction material on both sides thereof. The means of attaching the flexible support to a single planar element may comprise hook and loop type fasteners, a pocket in the pelvic support into which the end of the planar element locates on any other conventional fastening means.

According to one embodiment of the invention the first and/or second planar element may comprise at least one inflatable chamber, the inflatable chamber giving the element its rigidity or semi-rigidity when inflated.

Specific embodiments of the invention will now be described, by way of example, with reference to the accompanying drawings in which:
Figure 1 shows a lower planar element of the invention;
Figure 2 shows an upper planar element of the invention;
Figure 3 shows the method of attaching a pelvic support the lower planar element of Figure 1;
Figure 4 shows the apparatus of the invention assembled with a pelvic support; and
Figure 5 to 7 show the steps of the device being used.

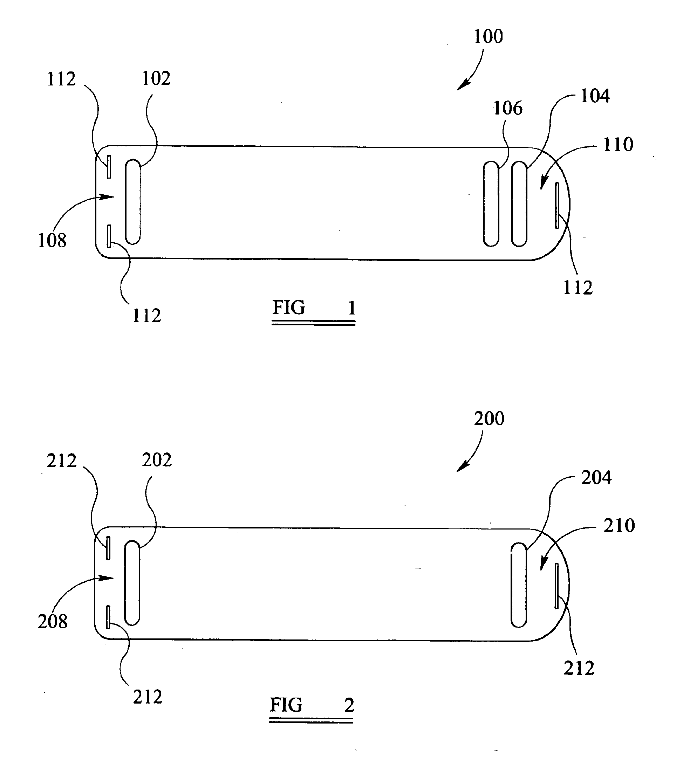

Referring to Figures 1 and 2, lower 100 and upper 200 planar elements are shown. Both the lower 100 and upper 200 elements are elongate in shape and have a length in the region of 750mm. The length may vary but in any case is sufficiently long so as to enable the elements 100, 200, in use, to pass under the pelvic region of a casualty and protrude either side thereof.

The elements 100, 200 each comprise a sheet of polypropylene material, which is preferably of a food grade or similar such that they can be passed through standard hot sterilisation process without compromising the material.

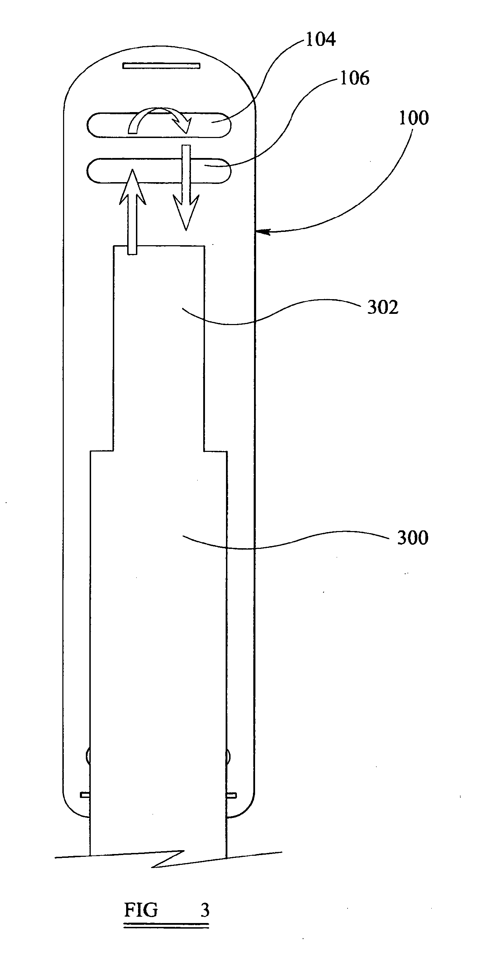

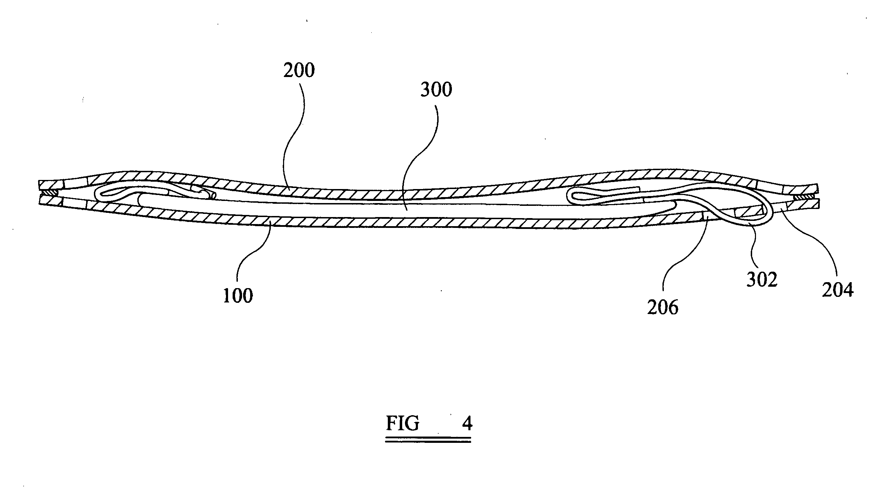

The lower panel 100 has three openings 102, 104, 106 therein. Openings 104 and 106 together form a means of, in use, attaching a pelvic support to the apparatus as described below in relation to Figure 3 and 4.

Openings 102, 104 of the lower panel form handles 108, 110 on the lower element and corresponding openings 202, 204 form corresponding handles 208, 210 on the upper element. In use the handles enable the assembled apparatus to be easily manipulated and used.

Both the lower 100 and upper 200 panels have hook and loop fasteners, for example Velcro® at either end, one part of the fasteners 112 being attached to the lower 100 element 100 and the other part of the fastener 212 being attached to the upper element 200. Although in the embodiment shown hook and loop type fasteners are shown at one end, in another preferred arrangement, not shown, the hook and loop fasteners are only attached at one end, that end being the end that is slid under the casualty. The other end is held together by the person deploying the pelvic support.

Referring now to Figures 3 and 4, in use a strap type pelvic support 300 is placed over the lower element 100 along its length on the side having the fasteners thereon. An end 302 of the pelvic support is passed through opening 106 and back through opening 104 such that it is threaded through the element 100. The end 302 of the pelvic support 300 is then folded back on itself such that it at least partially overlays itself. The upper element 200 is then attached to the lower element 100 by means of the hoop and loop fasteners such that the pelvic support 300 is sandwiched between the two planar elements.

Although this arrangement is described, an equally, and somewhat simpler arrangement can be achieved by merely sandwiching the pelvic support between the two planar elements without physically attaching it to either one of them. In this arrangement friction between the two boards and the pelvic support maintain it in place while it is slid under the casualty. This type of arrangement is especially beneficial as it reduced the time taken to deploy the pelvic support. In such an arrangement the means for securing the pelvic support adjacent the element is compression/friction between the two elements 100, 200 and the pelvic support 300

Once the upper and lower elements have been attached to one another, flexure of the elements creates a small force between them. Friction between the overlying surfaces of the end 302 of the pelvic support 300, and between the end 302 of the pelvic support and the lower element 100 where it passes therethrough, retain the pelvic support sufficiently securely such that the assembled apparatus can be deployed without the pelvic support coming free. The assembled apparatus is then ready for deployment.

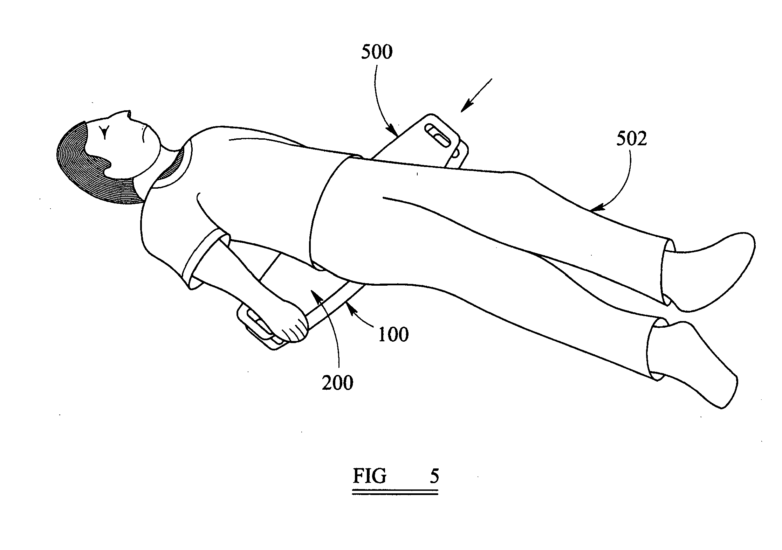

Referring to Figures 5 to 7 the assembled apparatus 500 is slid under the casualty 502 in the pelvic region (Figure 5). As the pelvic support, which is flexible in nature, is sandwiched between the two elements of the apparatus, which are semi-rigid in nature, the assembled apparatus, including the pelvic support can easily be slid under the casualty with minimum need for disturbance. The polypropylene material of the exterior of the apparatus has a low coefficient of friction and as such can relatively easily be slid under the casualty. By gently lifting the garments on one side of the patient so as to position the nose of the apparatus in position, it can be slid under the patient in one smooth motion and, although it is seen as beneficial to have more than one medical professional attending such a casualty, the operation can be carried out by a single operative. Without the use of the apparatus of the invention, a strap type pelvic support can not be simply slid under the casualty as, not only does it not have sufficient rigidity, but the nature of the material of such supports typically has a fabric surface and as such will catch on the clothes of the casualty requiring the casualty to be moved a considerable amount, and possibly lifted in the pelvic region, to enable attending medical professionals to fit the support.

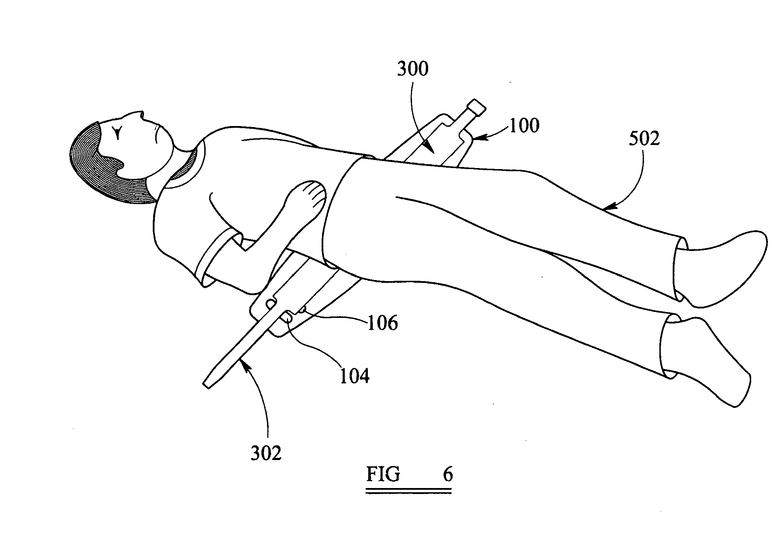

Once the assembled apparatus 500 is in situ under the casualty, the upper element 200 is separated from the lower element 100 and is slid out from between the casualty 502 and the pelvic support 300, leaving the pelvic support 300 adjacent the casualty 502 (Figure 6). Where the hook and loop type fasteners are only provided on the nose end of the apparatus this is facilitated as, if pulled out from the nose end there is nothing on the surface of the upper element to snag on the pelvic support, thereby facilitating its removal. The pelvic support 300 is then detached from the lower element 100 by taking the end 302 from the holes 104, 106 so that it can be wrapped around the pelvis of the casualty 500 and secured in place so as to support, and restrain movement of, the pelvic area. The pelvic support 300 is of a know type, for example the SAM Pelvic Support and functions in its known manner. Once the support 300 is fitted, the casualty 500 may then be more safely moved with the pelvic support in place.

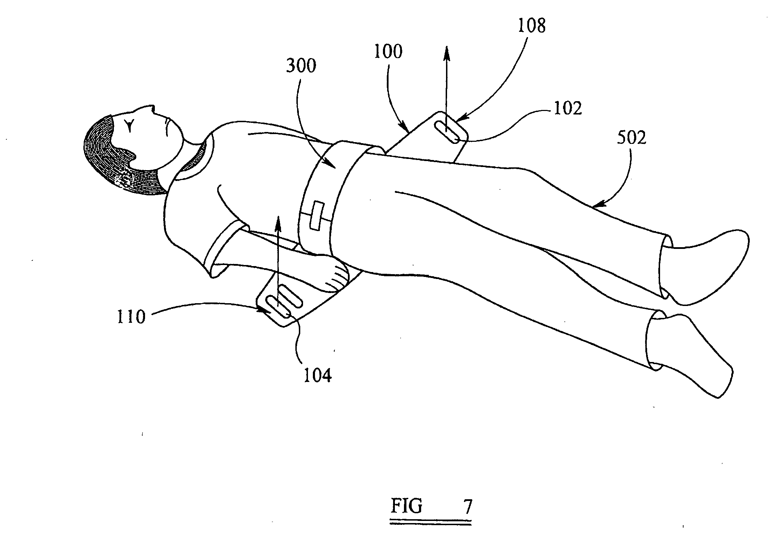

The lower element 100 may also be removed from beneath the casualty 500 by sliding it out or may be left in place, below the casualty until the casualty 500 has been moved. Alternatively, the lower element 100 may be gripped by the handles 108, 110 at either end thereof and used to assist in supporting the weight of the casualty 500 as they are moved or lifted. In particular, using the lower element 100 in this manner will give support to the pelvis and assist in maintaining the pelvis in a relatively sable manner as the casualty 500 is moved (Figure 7).

Another benefit of the present invention is the reduced time for application of pelvic supports which can, in severe injuries mean that the casualty can be moved into a hospital in a shorter time, often increasing chance of and/or time taken for recovery.

A particularly important application is in the removal of casualties from vehicles after motor accidents. Usually the fire service wants to remove casualties from cars in the shortest possible time due to the risk of fire etc from any seeping fuel. Furthermore, it is often necessary to remove casualties to access other casualties who may be trapped in the vehicle. Moving casualties out of cars is not an easy task and usually requires extensive manhandling of the casualty which causes disturbances to the pelvic region. It is currently virtually impossible to pass a pelvic support between a casualty and a car seat without significantly disturbing the casualty. With the present invention, especially when a semi-rigid material is used that has some flexure in it, the apparatus, with the pelvic support attached, can simply be slid in between the patient and the car seat. This enables the attending paramedics or fire service personnel to quickly stabilise a casualty in a vehicle before removing them therefrom.

Although the above specific embodiment utilised two elements 100, 200 it will be appreciated that the invention can be practiced with just one element providing it gives sufficient rigidity to the pelvic support to enable it to be slid under a patient. In such an embodiment the element and support may be inserted under the patient in either orientation (support above or support below the element). Optionally the support may have a low friction surface such that when the support is adjacent the element the combined assembly has a low friction surface adjacent the ground and adjacent the casualty.

The various features above described above with respect to the device with two elements may, where appropriate, be used with an applicator having only one element

## Claims

1. A pelvic support application apparatus adapted to position a flexible pelvic support (300) adjacent a patient/casualty comprising
at least a first semi-rigid or rigid elongate planar element (100),
**characterised in that** it comprises: a means for releasably attaching a flexible pelvic support (300) adjacent and to said at least one planar element (300) such that, in use the planar element and pelvic support are adapted to be slid between a person and a surface against which the person is supported wherein
the means for attaching the pelvic support (300) comprises two openings (104, 106) in one end of said at least one planar element (100) such that, in use, the pelvic support (300) is adapted to be passed outwardly through one opening, back in the other opening, and back on itself such that it at least partially overlays itself.

2. The apparatus according to claim 1 further comprising a second elongate planar element (200) releasably attachable to the first elongate planar element (100).

3. The apparatus according to claim 2 wherein the second elongate element (200) is rigid or semi-rigid.

4. The apparatus according to claim 2 or claim 3 wherein, in use, the pelvic support (300) is sandwiched between said first (100) and second (200) planar elements.

5. The apparatus according to any preceding claim wherein the first planar element (100) has a low coefficient of friction.

6. The apparatus according to any one of claims 2 to 4 wherein the first and/or second planar element (100, 200) has a low coefficient of friction.

7. The apparatus according to claim 5 or claim 6 wherein the first planar element (100) is made of polypropylene.

8. The apparatus according to any preceding claim in which the first planar element (100) has handles at either end thereof.

9. The apparatus according to any one of claims 2 to 4 wherein the first (100) and/or second planar element (200) has handles at either end thereof.

10. The apparatus according to claim 8 or claim 9 wherein one of the two openings (104, 106) in the end of said first planar element (100) also provides the handle.

11. The apparatus according to claim 2 further comprising attachment means for attaching the first (100) and second (200) planar elements to one another.

12. The apparatus according to claim 11 wherein the means for attaching the first (100) and second (200) planar elements to one another comprises a multiple hook and loop type fastener.

13. The apparatus according to claim 11 or claim 12 wherein the means for attaching the first (100) and second (200) planar elements to one another is located only at one end of the planar elements.

14. The apparatus according to claim 1 further comprising a pelvic support (300).

15. The apparatus according to claim 14 wherein the external side of the pelvic support (300), when secured adjacent the planar element (100), comprises a low friction material.

## Patentansprüche

1. Beckenstützen-Anwendungsvorrichtung, die dafür eingerichtet ist, eine flexible Beckenstütze (300) angrenzend an einen Patienten/Verletzten zu positionieren, wobei die Vorrichtung Folgendes umfasst:
wenigstens ein erstes halbstarres oder starres längliches ebenes Element (100), **dadurch gekennzeichnet, dass** es Folgendes umfasst:
ein Mittel zum lösbaren Befestigen einer flexiblen Beckenstütze (300) angrenzend an und an dem wenigstens einen ebenen Element (100) derart, dass bei Anwendung das ebene Element und die Beckenstütze dafür eingerichtet sind, zwischen eine Person und eine Oberfläche, gegen welche die Person gestützt wird, geschoben zu werden, wobei
die Mittel zum Befestigen der Beckenstütze (300) zwei Öffnungen (104, 106) in einem Ende des wenigstens einen ebenen Elements (100) umfassen derart, dass bei Anwendung die Beckenstütze (300) dafür eingerichtet ist, durch die eine Öffnung nach außen, in die andere Öffnung zurück und auf sich selbst zurück geführt zu werden, so dass sie sich wenigstens teilweise selbst überlagert.

2. Vorrichtung nach Anspruch 1, die ferner ein zweites längliches ebenes Element (200) umfasst, das lösbar an dem ersten länglichen ebenen Element (100) befestigt werden kann.

3. Vorrichtung nach Anspruch 2, wobei das zweite längliche Element (200) starr oder halbstarr ist.

4. Vorrichtung nach Anspruch 2 oder Anspruch 3, wobei bei Anwendung die Beckenstütze (300) zwischen dem ersten (100) und dem zweiten (200) ebenen Element eingeklemmt ist.

5. Vorrichtung nach einem der vorhergehenden Ansprüche, wobei das erste ebene Element (100) einen niedrigen Reibungskoeffizienten hat.

6. Vorrichtung nach einem der Ansprüche 2 bis 4, wobei das erste und/oder das zweite ebene Element (100, 200) einen niedrigen Reibungskoeffizienten hat.

7. Vorrichtung nach Anspruch 5 oder Anspruch 6, wobei das erste ebene Element (100) aus Polypropylen hergestellt ist.

8. Vorrichtung nach einem der vorhergehenden Ansprüche, wobei das erste ebene Element (100) an beiden Enden desselben Griffe hat.

9. Vorrichtung nach einem der Ansprüche 2 bis 4, wobei das erste und/oder das zweite ebene Element (100, 200) an beiden Enden desselben Griffe hat.

10. Vorrichtung nach Anspruch 8 oder Anspruch 9, wobei eine der zwei Öffnungen (104, 106) in dem Ende des ersten ebenen Elements (100) ebenfalls den Griff bereitstellt.

11. Vorrichtung nach Anspruch 2, die ferner Mittel zum Befestigen des ersten (100) und des zweiten (200) ebenen Elements aneinander umfasst.

12. Vorrichtung nach Anspruch 11, wobei die Mittel zum Befestigen des ersten (100) und des zweiten (200) ebenen Elements aneinander einen mehrfachen Klettverschluss umfassen.

13. Vorrichtung nach Anspruch 11 oder Anspruch 12, wobei die Mittel zum Befestigen des ersten (100) und des zweiten (200) ebenen Elements aneinander nur an einem Ende der ebenen Elemente angeordnet sind.

14. Vorrichtung nach Anspruch 1, die ferner eine Beckenstütze (300) umfasst.

15. Vorrichtung nach Anspruch 14, wobei die äußere Seite der Beckenstütze (300), wenn sie angrenzend an das erste ebene Element (100) befestigt ist, einen reibungsarmen Werkstoff umfasst.

## Revendications

1. Dispositif d'application d'un support pelvien, adapté pour positionner un support pelvien flexible (300) près d'un patient/d'un blessé, comprenant :
au moins un premier élément plan allongé semi-rigide ou rigide (100), **caractérisé en ce qu'**il comprend :
un moyen pour fixer de manière amovible un support pelvien flexible (300) près dudit au moins un élément plan (100) et sur celui-ci, de sorte qu'en service, l'élément plan et le support pelvien sont adaptés pour être glissés entre une personne et une surface contre laquelle la personne est supportée, dans lequel
le moyen destiné à fixer le support pelvien (300) comprend deux ouvertures (104, 106) dans une extrémité dudit au moins un élément plan (100), de sorte qu'en service, le support pelvien (300) est adapté pour être passé vers l'extérieur à travers une ouverture, ramené dans l'autre ouverture et ramené sur lui-même de manière à être en partie superposé à lui-même.

2. Dispositif selon la revendication 1, comprenant en outre un deuxième élément plan allongé (200), pouvant être fixé de manière amovible sur le premier élément plan allongé (100).

3. Dispositif selon la revendication 2, dans lequel le deuxième élément allongé (200) est rigide ou semi-rigide.

4. Dispositif selon les revendications 2 ou 3, dans lequel en service, le support pelvien (300) est agencé en sandwich entre lesdits premier (100) et deuxième (200) éléments plans.

5. Dispositif selon l'une quelconque des revendications précédentes, dans lequel le premier élément plan (100) présente un coefficient de frottement réduit.

6. Dispositif selon l'une quelconque des revendications 2 à 4, dans lequel le premier et/ou le deuxième élément plan (100, 200) présente un coefficient de frottement réduit.

7. Dispositif selon les revendications 5 ou 6, dans lequel le premier élément plan (100) et composé de polypropylène.

8. Dispositif selon l'une quelconque des revendications précédentes, dans lequel le premier élément plan (100) comporte des poignées au niveau de chacune de ses extrémités.

9. Dispositif selon l'une quelconque des revendications 2 à 4, dans lequel le premier (100) et/ou le deuxième élément plan (200) comporte des poignées au niveau de chacune de ses extrémités.

10. Dispositif selon les revendications 8 ou 9, dans lequel une des deux ouvertures (104, 106) dans l'extrémité dudit premier élément plan (100) établit aussi la poignée.

11. Dispositif selon la revendication 2, comprenant en outre un moyen de fixation pour fixer les premier (100) et deuxième (200) éléments plans l'un à l'autre.

12. Dispositif selon la revendication 11, dans lequel le moyen destiné à fixer les premier (100) et deuxième (200) éléments plans l'un à l'autre comprend un moyen de fixation du type à boucles et crochets multiples.

13. Dispositif selon les revendications 11 ou 12, dans lequel le moyen destiné à fixer les premier (100) et deuxième (200) éléments plans l'un à l'autre est agencé uniquement au niveau d'une extrémité des éléments plans.

14. Dispositif selon la revendication 1, comprenant en outre un support pelvien (300).

15. Dispositif selon la revendication 14, dans lequel le côté externe du support pelvien (300) fixé près de l'élément plan (100) comprend un matériau à coefficient de frottement réduit.
